# EUROPEAN PATENT SPECIFICATION

(11) **EP 2 556 697 B1**
(45) Date of publication and mention of the grant of the patent: **16.05.2018**
(21) Application number: 11714112.7
(22) Date of filing: 15.03.2011
(51) Int. Cl.: H04W 28/16, A61B 5/00, A61N 1/372, H04W 16/16, H04W 72/08, H04W 84/22

(54) **CENTRALIZED DYNAMIC CHANNEL ALLOCATION FOR MEDICAL BODY AREA NETWORKS**
ZENTRALISIERTE DYNAMISCHE KANALZUWEISUNG FÜR MEDIZINISCHE WBAN
ALLOCATION DE CANAL DYNAMIQUE CENTRALISÉE POUR DES RÉSEAUX CORPORELS MÉDICAUX

(30) Priority: 06.04.2010 US 321164 P
(43) Date of publication of application: 13.02.2013
(73) Proprietor: Koninklijke Philips N.V., 5656 AE Eindhoven (NL)
(72) Inventor: WANG, Dong, Briarcliff Manor, New York 10510-8001 (US); ZHAI, Hongqiang, Briarcliff Manor, New York 10510-8001 (US); GHOSH, Monisha, Briarcliff Manor, New York 10510-8001 (US)
(74) Representative: Damen, Daniel Martijn
(86) International application number: PCT/IB2011/051080
(87) International publication number: WO 2011/124995

(56) References cited:
- EP-A1- 1 732 338
- EP-A2- 2 083 594
- NATARAJAN A ET AL: "Inter-User Interference in Body Sensor Networks: Preliminary Investigation and an Infrastructure-Based Solution", WEARABLE AND IMPLANTABLE BODY SENSOR NETWORKS, 2009. BSN 2009. SIXTH INTERNATIONAL WORKSHOP ON, IEEE, PISCATAWAY, NJ, USA, 3 June 2009 (2009-06-03), pages 35-40, XP031522356, ISBN: 978-0-7695-3644-6

## Description

The following relates to the medical monitoring arts and related arts.

A medical body area network (MBAN) replaces the tangle of cables tethering hospital patients to their bedside monitoring units with wireless connections. This provides low-cost wireless patient monitoring (PM) without the inconvenience and safety hazards posed by wired connections, which can trip medical personnel or can become detached so as to lose medical data. In the MBAN approach, multiple low cost sensors are attached at different locations on or around a patient, and these sensors take readings of patient physiological information such as patient temperature, pulse, blood glucose level, electrocardiographic (ECG) data, or so forth. The sensors are coordinated by at least one proximate hub or gateway device to form the MBAN. The hub or gateway device communicates with the sensors using embedded short-range wireless communication radios, for example conforming with an IEEE 802.15.4 (Zigbee) short-range wireless communication protocol. Information collected by the sensors is transmitted to the hub or gateway device through the short-range wireless communication of the MBAN, thus eliminating the need for cables. The hub or gateway device communicates the collected patient data to a central patient monitoring (PM) station via a wired or wireless longer-range link for centralized processing, display and storage. The longer-range network may, for example, include wired Ethernet and/or a wireless protocol such as Wi-Fi or some proprietary wireless network protocol. The PM station may, for example, include an electronic patient record database, display devices located at a nurse's station or elsewhere in the medical facility, or so forth.

MBAN monitoring acquires patient physiological parameters. Depending upon the type of parameter and the state of the patient, the acquired data may range from important (for example, in the case of monitoring of a healthy patient undergoing a fitness regimen) to life-critical (for example, in the case of a critically ill patient in an intensive care unit). In general, there is a strict reliability requirement on the MBAN wireless links due to the medical content of the data.

Short-range wireless communication networks, such as MBAN systems, tend to be susceptible to interference. The spatially distributed nature and typically *ad hoc* formation of short-range networks can lead to substantial spatial overlap of different short range networks. The number of short-range communication channels allocated for short range communication systems is also typically restricted by government regulation, network type, or other factors. The combination of overlapping short-range networks and limited spectral space (or number of channels) can result in collisions between transmissions of different short range networks. These networks can also be susceptible to radio frequency interference (RFI) from other sources, including sources that are not similar to short-range network systems.

In EP 2 083 594 A2 a method and system for interference mitigation in a wireless communication system formed by multiple radio access networks is presented. A wireless communication system comprises multiple radio access networks (RANs), which at least partly share the same frequency spectrum. Each RAN is provided with a gateway for managing the network. Spectrum sharing in the system is achieved through a hierarchy of processes including long-term spectrum assignment, short-term spectrum assignment, and dynamic channel allocation, the latter process assigning sub-channels to base stations in each RAN.

In de Silva et al., "Inter-User Interference in Body Sensor Networks: Preliminary Investigation and an Infrastructure-Based Solution, Body Sensor Networks 2009, a study and a solution approach to inter-user interference in Body Sensor Networks are presented. Inter-user interference is the interference in desired communication when several Body Sensor Networks (BSNs) operate in the same vicinity. As BSN users congregate in an area, interference due to concurrently communicating BSNs will increase, resulting in degradation in performance. There is also proposed a system to mitigate the adverse effects of inter-user interference. The solution uses a fixed network infrastructure to monitor and identify BSNs that are likely to interfere with each other. The network then recommends changes to the BSN protocol to reduce interference between them. It is known to employ frequency agility mechanisms to mitigate radio frequency interference (RFI) in short range networks. For example, in IEEE 802.15.4 (Zigbee) systems clear channel assessment (CCA) may be employed to identify a clear channel for communication and to avoid communicating on a busy channel or on a channel that is susceptible to RFI from other sources. In the Bluetooth™ system, random frequency hopping is used to mitigate the possible interference from other co-existing networks. Other approaches include direct sequence spectrum spreading (DSSS) and listen-before-talk protocols. A complementary approach is to perform error checking of the communicated data, for example employing checksum testing or so forth. If the communicated data fails the error checking it can be re-transmitted to ensure accuracy.

These techniques are generally effective for short range communication network applications which can tolerate some error and/or transmission delay. Different MBAN systems, depending on their applications, usually have different tolerance to transmission errors and delay. MBAN systems for fitness or wellbeing applications usually are able to tolerate such transmission errors and delay. However, MBAN systems for high-acuity monitoring usually carry life-critical medical data and thus have little or no error tolerance, and also are not amenable to transmission delays such as may be introduced by retransmission. Transmission delays are problematic for such MBAN systems because delays in communication of life-critical data can delay detection of the onset of a life-threatening condition. Moreover, the sensor nodes of an MBAN system are preferably small (for patient comfort) and of minimal complexity (to enhance reliability and reduce manufacturing cost). The sensor nodes therefore typically have limited on-board data buffering, and so a continuously monitored life-critical parameter such as ECG data must be expeditiously transmitted off the sensor node to avoid losing the data.

The following provides new and improved apparatuses and methods which overcome the above-referenced problems and others.

In accordance with one disclosed aspect, a medical system comprises: a plurality of medical body area network (MBAN) systems, each MBAN system comprising a plurality of network nodes, said nodes arranged to acquire patient data and intercommunicate via short range wireless communication; a central network communicating with the MBAN systems via longer range communication that is of longer range than the short range wireless communication; and a central frequency agility sub-system configured to communicate with the MBAN systems, the central frequency agility sub-system receiving current channel quality information for a plurality of available channels for the short range wireless communication and allocating the MBAN systems amongst the available channels based at least on the received channel quality information and on radio inference ratings being indicative for the likelihood of non-MBAN radio frequency interference on each of the plurality of available channels and on criticality of the patient data acquired by each of the plurality of MBAN systems.

In accordance with another disclosed aspect, a method for channel allocation in a medical system comprising a plurality of MBAN systems, each MBAN system comprising a plurality of network nodes, said nodes arranged to acquire patient data and intercommunicate via short-range wireless communication and a central network arranged to communicate with the MBAN systems via longer range communication that is of longer range than the short-range wireless communication is provided, said method comprising: collecting current channel quality information for a plurality of channels usable by the plurality MBAN, systems for short-range communication amongst network nodes of the MBAN systems; and allocating the MBAN systems amongst the channels based at least on the collected current channel quality information and on radio frequency interference ratings being indicative for the likelihood of non-MBAN radio frequency interference on each of the plurality of available channels and on criticality of the patient data acquired by each of the plurality of MBAN systems. One advantage resides in safe co-existence of multiple MBAN systems which may overlap in space.

Another advantage resides in reduced or eliminated likelihood of transmission delays within or from an MBAN system.

Another advantage resides in reduced or eliminated likelihood of loss of critical medical data acquired by an MBAN system.

Another advantage resides in principled allocation of short-range communication channels of varying quality to MBAN systems in accordance with the criticality of data acquired by the various MBAN systems.

Further advantages will be apparent to those of ordinary skill in the art upon reading and understanding the following detailed description.
FIGURE 1 diagrammatically illustrates a medical body area network (MBAN) system in the context of a medical environment including a central frequency agility sub-system as disclosed herein.
FIGURE 2 diagrammatically illustrates an ordered list of available channels suitably generated by the central frequency agility sub-system of FIGURE 1.
FIGURE 3 diagrammatically illustrates initial processing flow in the central frequency agility sub-system of FIGURE 1 and in the MBAN system of FIGURE 1 as these systems are initialized.
FIGURE 4 diagrammatically illustrates processing flow in the central frequency agility sub-system of FIGURE 1 responsive to a request for allocation of a communication channel for a new MBAN system.

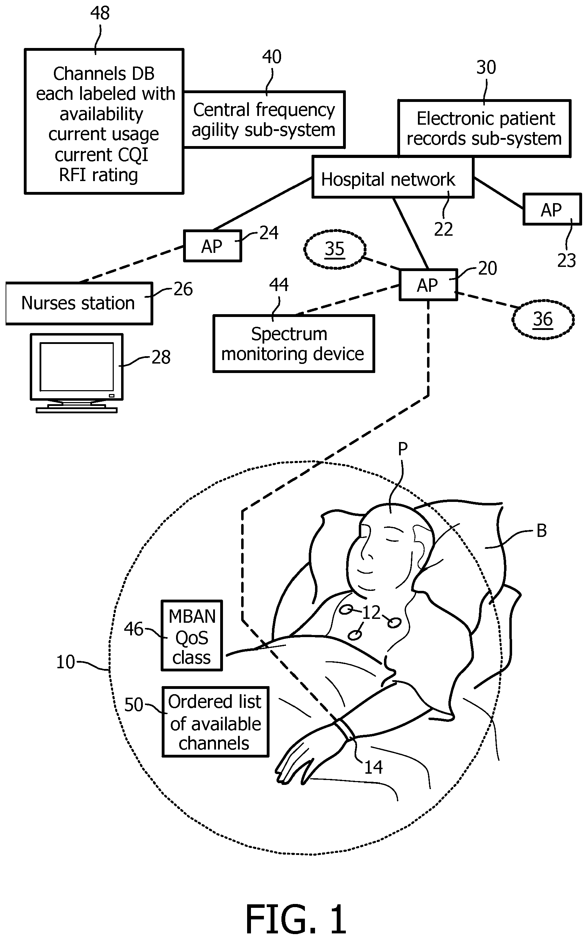

With reference to FIGURE 1, a medical body area network (MBAN) **10** includes a plurality of network nodes **12, 14.** At least one of the network nodes **12, 14** serves as a hub device **14.** The network nodes **12** communicate with the hub device **14** via a short-range wireless communication protocol. The MBAN **10** is also sometimes referred to in the relevant literature by other equivalent terms, such as a body area network (BAN), a body sensor network (BSN), a personal area network (PAN), a mobile ad hoc network (MANET), or so forth - the term medical body area network (MBAN) **10** is to be understood as encompassing these various alternative terms.

The illustrative MBAN **10** includes four illustrative network nodes **12, 14** including the hub device **14;** however, the number of network nodes can be one, two, three, four, five, six, or more, and moreover the number of network nodes may in some embodiments increase or decrease in an *ad hoc* fashion as sensor nodes are added or removed from the network to add or remove medical monitoring capability. The network nodes **12** are typically sensor nodes that acquire physiological parameters such as heart rate, respiration rate, electrocardiographic (ECG) data, or so forth; however, it is also contemplated for one or more of the network nodes to perform other functions such as controlled delivery of a therapeutic drug via a skin patch or intravenous connection, performing cardiac pacemaking functionality, or so forth. A single network node may perform one or more functions. The illustrative network nodes **12** are disposed on the exterior of an associated patient **P;** however, more generally the network nodes may be disposed on the patient, or in the patient (for example, a network node may take the form of an implanted device), or proximate to the patient within the communication range of the short-range communication protocol (for example, a network node may take the form of a device mounted on an intravenous infusion pump (not shown) mounted on a pole that is kept near the patient, and in this case the monitored patient data may include information such as the intravenous fluid flow rate). It is sometimes desirable for the network nodes to be made as small as practicable to promote patient comfort, and to be of low complexity to enhance reliability - accordingly, such network nodes **12** are typically low-power devices (to keep the battery or other electrical power supply small) and may have limited on-board data storage or data buffering. As a consequence, the network nodes **12** should be in continuous or nearly continuous short-range wireless communication with the hub device **14** in order to expeditiously convey acquired patient data to the hub device **14** without overflowing the data buffer.

The hub device **14** (also sometimes referred to in the relevant literature by other equivalent terms, such as "gateway device" or "hub node") coordinates operation of the MBAN **10** by collecting (via the Zigbee, Bluetooth™, or other short-range wireless communication protocol) patient data acquired by the sensors of the network nodes **12** and transmitting the collected data away from the MBAN **10** via a longer range communication protocol. The short-range wireless communication protocol preferably has a relatively short operational range of a few tens of meters, a few meters, or less, and in some embodiments suitably employs an IEEE 802.15.4 (Zigbee) short-range wireless communication protocol or a variant thereof, or a Bluetooth™ short-range wireless communication protocol or a variant thereof. Both Bluetooth™ and Zigbee operate in a frequency spectrum of around 2.4-2.5 GHz. Although Bluetooth™ and Zigbee are suitable embodiments for the short-range wireless communication, other short-range communication protocols, including proprietary communication protocols, are also contemplated. Moreover, the short-range wireless communication can operate at other frequencies besides the 2.4-2.5 GHz range, such as ranges in the hundreds of megahertz, gigahertz, tens-of-gigahertz, or other ranges. The short-range communication protocol should have a sufficient range for the hub device **14** to communicate reliably with all network nodes **12** of the MBAN system **10.** In FIGURE 1, this short-range wireless communication range is diagrammatically indicated by the dotted oval used to delineate the MBAN system **10.** The short-range wireless communication is typically two-way, so that the network nodes **12** can communicate information (e.g., patient data, network node status, or so forth) to the hub device **14;** and the hub device **14** can communicate information (e.g., commands, control data in the case of a therapeutic network node, or so forth) to the network nodes **12.** The illustrative hub device **14** is a wrist-mounted device; however, the hub device can be otherwise mounted to the patient, for example as a necklace device, adhesively glued device, or so forth. It is also contemplated for the hub device to be mounted elsewhere proximate to the patent, such as being integrated with an intravenous infusion pump (not shown) mounted on a pole that is kept near the patient.

The hub device **14** also includes a transceiver (not shown) providing the longer-range communication capability to communicate data off the MBAN system **10.** In the illustrative example of FIGURE 1, the hub device **14** wirelessly communicates with an access point (AP) **20** of a hospital network **22.** The illustrative AP **20** is a wireless access point that communicates wirelessly with the hub device **14.** In the illustrative embodiment the hospital network **22** also includes additional access points, such as illustrative access points AP **23** and AP **24,** that are distributed throughout the hospital or other medical facility. To provide further illustration, a nurses' station **26** is diagrammatically indicated, which is in wireless communication with the AP **24** and includes a display monitor **28** that may, for example, be used to display medical data for the patient **P** that are acquired by the MBAN system **10** and communicated to the nurses' station **26** via the path comprising the AP **20,** the hospital network **22,** and the AP **24.** By way of another illustrative example, the hospital network **22** may provide access with an electronic patient records sub-system **30** in which is stored medical data for the patient **P** that are acquired by the MBAN system **10** and communicated to the electronic patient records sub-system **30** via the path comprising the AP **20** and the hospital network **22.** The illustrative longer-range communication between the hub device **14** and the AP **20** is wireless, as diagrammatically indicated in FIGURE 1 by a dashed connecting line. (Similarly, wireless communication between the AP **24** and the nurses' station **26** is indicated by a dashed connecting line). In some suitable embodiments, the longer-range wireless communication is suitably a WiFi communication link conforming with an IEEE 802.11 wireless communication protocol or a variant thereof. However, other wireless communication protocols can be used for the longer-range communication, such as another type of wireless medical telemetry system (WMTS). Moreover, the longer range communication can be a wired communication such as a wired Ethernet link (in which case the hub device includes at least one cable providing the wired longer range communication link).

The longer range communication is longer range as compared with the short-range communication between the network nodes **12** and the hub device **14.** For example, while the short-range communication range may be of order a few tens of centimeters, a few meters, or at most perhaps a few tens of meters, the longer range communication typically encompasses a substantial portion of the hospital or other medical facility through the use of multiple access points **20, 23, 24** or, equivalently, multiple Ethernet jacks distributed throughout the hospital, in the case of a wired longer-range communication.

The longer-range communication, if wireless, requires more power than the short-range communication - accordingly, the hub device **14** includes a battery or other power source sufficient to operate the longer-range communication transceiver. Alternatively, the hub device **14** may include a wired electrical power connection. The hub device **14** also typically includes sufficient on-board storage so that it can buffer a substantial amount of patient data in the event that communication with the AP **20** is interrupted for some time interval. In the illustrative case of wireless longer-range communication, it is also to be understood that if the patient **P** moves out of range of the AP **20** and into range of another AP (for example, AP **23** or AP **24**) then the IEEE 802.11 or other wireless communication protocol employed by the hospital network **22** (including its wireless access points **20, 23, 24**) provides for the wireless link to shift from AP **20** to the newly proximate AP. In this regard, although the patient **P** is illustrated as lying in a bed **B,** more generally it is contemplated for the patient **P** to be ambulatory and to variously move into and out of range of the various access points **20, 23, 24.** As the patient **P** thus moves, the MBAN **10** including the network nodes **12** and the hub device **14** moves together with the patient **P.**

In the MBAN **10,** the network nodes **12** communicate with the hub device **14** via the short-range wireless communication. However, it is also contemplated for various pairs or groups of the network nodes **12** to also intercommunicate directly (that is, without using the hub device **14** as an intermediary) via the short-range wireless communication. This may be useful, for example, to coordinate the activities of two or more network nodes in time. Moreover, the hub device **14** may provide additional functionality - for example, the hub device **14** may also be a network node that includes one or more sensors for measuring physiological parameters. Still further, while the single hub device **14** is illustrated, it is contemplated for the coordinating functionality (e.g. data collection from from the network nodes **12** and offloading of the collected data via the longer range wireless communication) to be embodied by two or more network nodes that cooperatively perform the coodinating tasks.

In illustrative FIGURE 1, only the single MBAN system **10** is illustrated in detail. However, it will be appreciated that more generally the hospital or other medical facility includes a plurality of patients, each having his or her own MBAN system. This is diagrammatically shown in FIGURE 1 by two additional MBAN systems **35, 36** also communicating with the AP **20** via the longer range wireless communication. More generally, the number of MBAN systems may be, by way of some illustrative exmaples: two, three, four, five, ten, twenty, or more. Indeed, it is even contemplated for a single patient to have two or more different, independently operating MBAN systems (not illustrated). In this environment, various MBAN systems can be expected to occasionally come into close proximity with one another, such that the ranges of the respective MBAN system short-range wireless communications overlap.

Moreover, the hospital or other medical facility typically has numerous sources of radio frequency interference (RFI), such as magnetic resonance (MR) imaging scanners, computed tomography (CT) systems, radiation therapy systems, wireless radios in celluar phones and computers, radio equipment for communicating with ambulances, emergency response helicopters, local police, fire, or other rescue workers, and so forth. As a consequence, the various MBAN systems should be allocated channels for their respective short-range communication in a way that substantially avoids non-MBAN RFI and in a way that substantially avoids interference between proximate MBAN systems.

It is disclosed herein to employ a central frequency agility (CFA) sub-system **40** for this purpose of assigning short-range communication channels to the MBAN systems in a way that substantially avoids non-MBAN RFI and in a way that substantially avoids interference between proximate MBAN systems. The CFA sub-system **40** does not employ distributed frequency agility techniques as is commonly the case for Zigbee, Bluetooth™, or other *ad hoc* short-range wireless communication networks, but rather centralizes the frequency agility processing. The centralized approach disclosed herein takes advantage of the existence of the centralized longer-range communication network **20, 22, 23, 24** which is available in the hospital or other medical facility and with which the MBAN systems are configured to communicate. By employing the centralized CFA sub-system **40** to implement frequency agility, it is possible to provide principled allocation of short-range communication channels of varying quality to MBAN systems in accordance with the criticality of data acquired by the various MBAN systems. For example, although all MBAN systems are expected to collect important medical data, some MBAN systems may collect life-critical medical data (or, as another example, may deliver life-sustaining therapeutic intervention); whereas, other MBAN systems may collect medical data from healthy patients who are undergoing wellness treatment such as a fitness regimen. By centralizing the frequency agility, it is possible to allocate those MBAN systems engaged in life-critical operations to the cleanest channels (in the sense of potential for RFI interference and current channel quality information), and to allocate less critical MBAN systems to lower-grade (but still acceptable) channels.

The CFA sub-system **40** operates over an area within which MBAN systems may reasonably be expected to interfere with one another and/or experience common non-MBAN RFI. For large medical facilities, such as a multifloor hospital, more than one CFA sub-system may be provided, with the CFA sub-systems distributed over the medical facility in order to provide frequency agility for the various regions of the facility. In one suitable approach, each AP **20, 23, 24** is provided with its own CFA sub-system - by way of illustrative example, the CFA sub-system **40** of FIGURE 1 is assumed to be associated with the AP **20** and to perform frequency agility for the MBAN systems **10, 35, 36** and for any other MBAN systems that communicate with the AP **20.** In such embodiments, the CFA sub-system **40** may be embodied by the processor of the AP **20** executing suitable software to implement the CFA sub-system **40.** Alternatively, the CFA sub-system **40** may be embodied by another processor communicating with the AP **20** via the hospital network **22.** Moreover, a single CFA sub-system may perform centralized frequency agility for MBAN systems communicating with two or more access points, or for other suitable groupings of the MBAN systems.

The CFA sub-system **40** receives as input current channel quality information (CQI) for the channels that are usable for the MBAN system short-range wireless communications. The current CQI information may be collected from various sources. In some embodiments, the MBAN systems **10, 35, 36** perform clear channel assessment (CCA) to generate the current CQI information. Additionally or alternatively, a dedicated spectrum monitoring device **44** (or a spatial distribution of such devices) may be provided to acquire the CQI information. The spectrum monitoring device **44** or devices are optionally AC powered so that they do not have batteries to be replaced or recharged. The CCA is suitably performed by energy detection (ED) or carrier sensing or other suitable CCA operations to generate in-band interference information for the channels. The CQI information may also include MBAN packet detection (for example, using a high-gain antenna) to acquire information about current activity on the channels, including estimation of transmission duty cycles. The CQI information may also include analysis of potential in-band interference to assess interference sources (e.g., 802.15.4, 802.11b/g, Bluetooth™, or so forth). The CQI information acquired by the MBAN systems **10, 35, 36** and/or the spectrum monitoring device **44** or devices are communicated to the CFA sub-system **40** via the longer range communication, so that the CQI information can be centrally collected at the CFA sub-system **40.**

The CFA sub-system **40** allocates the MBAN systems **10, 35, 36** amongst the available channels based at least on the received current CQI information. The allocation may also be based on other information, such as an RFI rating for each channel which indicates the likelihood of experiencing non-MBAN interference on that channel, and a quality of service (QoS) classification for the MBAN systems **10, 35, 36.** The latter information, if available, is used to bias the allocations toward assigning channels with better current CQI (and, optionally, RFI ratings indicative of lower likelihood of RFI) to MBAN systems having higher QoS classifications.

For example, in an illustrative MBAN QoS classification scheme, there are M classifications, with the highest QoS class (i.e., Class 1) being reserved for MBAN systems engaged in life-critical applications, and the lowest QoS class (i.e., Class M) used for non-critical applications such as fitness monitoring. The QoS class of an MBAN system can be assigned by a physician, nurse, or other medical personnel when the MBAN system is created. Additionally or alternatively, the QoS class of an MBAN system can be assigned automatically based on the application running on the MBAN system. In the latter case, the MBAN system is suitably assigned its class based on the most critical application being performed by the MBAN system. To diagrammatically illustrate, FIGURE 1 diagrammatically shows an MBAN QoS class **46** assigned to the MBAN system **10.** (It is to be understood that the other MBAN systems **35, 36** each also have an assigned MBAN QoS class).

The channels are also optionally assigned RFI ratings. These ratings are distinct from the current CQI for the channel because the RFI rating is not based on current measurements or on MBAN usage, but rather is based on the likelihood of non-MBAN RFI occurring on the channel. For example, in one suitable RFI rating scheme, there are 1,...,N RFI rating levels with RFI rating Level 1 assigned to channels with the lowest likelihood of non-MBAN RFI and Level N assigned to channels with the highest likelihood of non-MBAN RFI. As a more specific example, the inner M-band channels, which are reserved specially for MBAN applications and are expected to have the smallest non-MBAN RFI, may be assigned RFI Level 1. Conversely, RFI Level N is for the MBAN channels that have the highest probability of being interfered by other wireless systems, and may for example include ISM channels that overlap with the ISM 2.4GHz Wi-Fi channel. In some embodiments, the MBAN RFI ratings are predefined and stored in a database accessible by the CFA sub-system **40.**

In the illustrative embodiment, the CFA sub-system **40** maintains a channels database **48** that lists, for each channel, its availability, its current usage (i.e., which MBAN systems are assigned to the channel and, at least in the case of shared channels, their duty cycles), the current CQI for the channel, and the channel RFI rating. The availability of a channel indicates whether the channel can be used by MBAN systems. A channel may be listed as unavailable for various reasons: its current CQI may be so poor that it cannot be used by MBAN systems; or the channel may be available for MBAN usage on a secondary basis and is currently in use by a primary non-MBAN user; or so forth. The channels database **48** can have various formats and can store various channel information in various ways. As an illustrative embodiment, the following table structure can be used:

| |
|---|
| ```
     Table {
            Field: channel_number,
                   the MBAN channel number
            Field: channel_rating
                   the channel RFI rating
            Field: channel_status:
                   'Idle' if no MBAN uses this channel, otherwise 'busy'
            Field: active_MBAN_list
                   This field is empty if channel_status is 'idle', otherwise it is a
                   sub-table, which includes the information of active MBANs
                   on the channel
                   Sub-table {
                          Field: MBAN_id,
                                 The MBAN id number
                          Field: MBAN_QoS_class
                          Field: Duty_cycle
                                 The aggregated duty cycle of this MBAN
                          Field: Relative_timing
                                 The relative timing to the AP device. This
                                 field is optional when a superframe
                                 structure is used for MBANs and
                                 inter-MBAN synchronization is
                                 utilized to improve the channel
                                 usage efficiency.
                                 }
             }
``` |

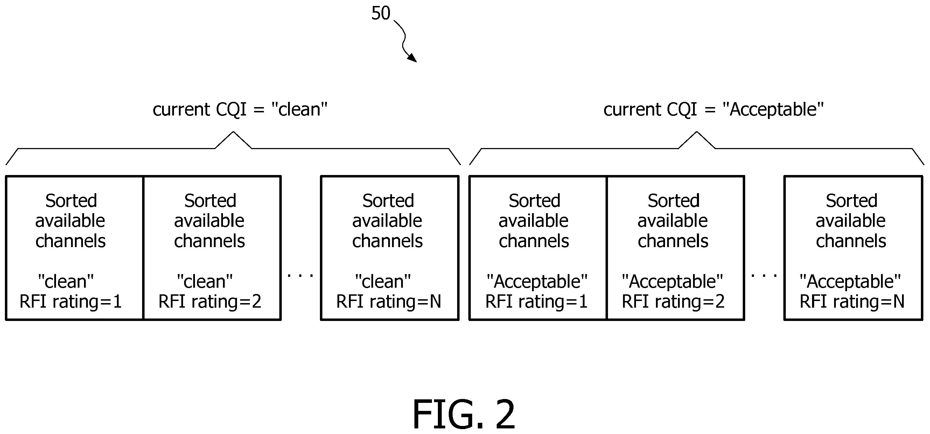

With continuing reference to FIGURE 1 and with further reference to FIGURE 2, to facilitate efficient operation of the MBAN systems **10, 35, 36,** in some embodiments an abridged copy of the channels database **48** is constructed by the CFA sub-system **40** and is communicated to the MBAN systems. In illustrative FIGURE 1, this is diagrammatically illustrated by an ordered list **50** of available channels that has been communicated to and is stored at the MBAN system **10.** (It is understood that copies of the ordered list **50** are also stored at each of the other MBAN systems **35, 36**)**.** FIGURE 2 diagrammatically shows the ordered list **50** in greater detail. The ordered list **50** of channels is sorted at least on the current CQI of the channel, and in the illustrative embodiment the ordered list **50** is secondarily sorted on the RFI rating of the channel. The ordered list **50** includes only those channels that are available for use in MBAN systems of at least one MBAN class. In the illustrative example: channel CQI class "Clean" is usable for MBAN systems of the highest MBAN Class 1 (e.g., life-critical applications) and are listed first in the ordered list **50;** channel CQI class "Acceptable" is usable for all MBAN systems except MBAN class 1, and is listed next in the ordered list **50;** and finally channel CQI class "Poor" is deemed unusable for any MBAN system of any type, and accordingly is not included in the ordered list **50.** The channels database **48** is updated and the ordered list **50** updated and resent to the MBAN systems **10, 35, 36** on a regular basis.

One approach for constructing the ordered list **50** is as follows. The input parameters include the measured channel CQI (in terms of non-MBAN-interference-plus-noise power) for all usable channels (including channels that may be listed as unavailable in the database **48**). The channel CQI is determined based on the channel qulity information measured by the MBAN systems **10, 35, 36** and/or by the optional dedicated spectrum monitoring device(s) **44.** The input parameters also optionally include the radio frequency spectrum used by current active non-MBAN wireless networks. This information could come from a database (not shown) accessible by the CFA sub-system **40,** for example via the hospital network **22.** Such a database may, for example, include empirical measurement data and/or information based on rated spectral RFI of electronic devices in the hospital. This information may also be embodied in the RFI ratings of the channels - for example, if a hospital MRI system is known to generate strong RFI at a particular channel, that channel may be given an RFI rating reflecting the expected high likelihood of experiencing RFI from the hospital MRI system. Another optional input is the RF spectrum to be protected. For example, if a band is allocated on a secondary basis and there are some primary users active in that band, then the current used RF spectrum by active primary users should not be allocated to any of the MBAN systems. This information may be generated by the CCA together with knowledge of the secondary allocation status of the channel for MBAN systems. The sorting algorithm is then suitably as follows. First, all the channels in the RF spectrum to be protected should be omitted from the ordered list **50.** (This suitably avoids having MBAN systems use spectrum currently used by primary users). Second, group the channels by RFI rating i, i = 1 to N. For the channels of each RFI rating, group the channels into three CQI groups: 'clean', 'acceptable', and 'dirty'. One way to do this is that if the non-MBAN-interference-plus-noise power is greater than a "dirty" threshold then label the channel as having a 'dirty' current CQI; else if the non-MBAN-interference-plus-noise power is less than a "clean" threshold (and the channel is not in the RF spectrum used by the current active non-MBAN wireless networks) then label it as 'clean'; else label it as 'acceptable'. Any channels that are labeled with a 'dirty' channel CQI are considered unavailable for allocation to MBAN networks and accordingly are omitted from the ordered list **50.** Finally, the remaining channels having channel CQI 'clean' or 'acceptable' are sorted based on the non-MBAN-interference-plus-noise power in an ascending order, and the results are combined to build up the ordered available channel list **50** as shown in FIGURE 2.

The ordered list **50** of available channels can be used in various ways by the MBAN system **10.** For example, in performing the CCA the MBAN system **10** optionally collects CQI information for only those channels listed in the ordered list **50.** This approach enhances efficiency by avoiding performing CCA on channels that are unavailable. As another application, in the event of RFI interference or collision on the currently allocated channel, the MBAN system **10** can refer to the ordered list **50** to identify a suitable 'clean' (or 'acceptable', in the case of MBAN QoS class **46** being non-life-critical) channel to which the MBAN system **50** can switch so as to avoid the RFI or collision. This local reallocation decision is then forwarded to the CFA sub-system **40** for entry in the channels database **48.** If the local reallocation decision is determined to be unacceptable by the CFA sub-system **40,** it can take suitable remedial action.

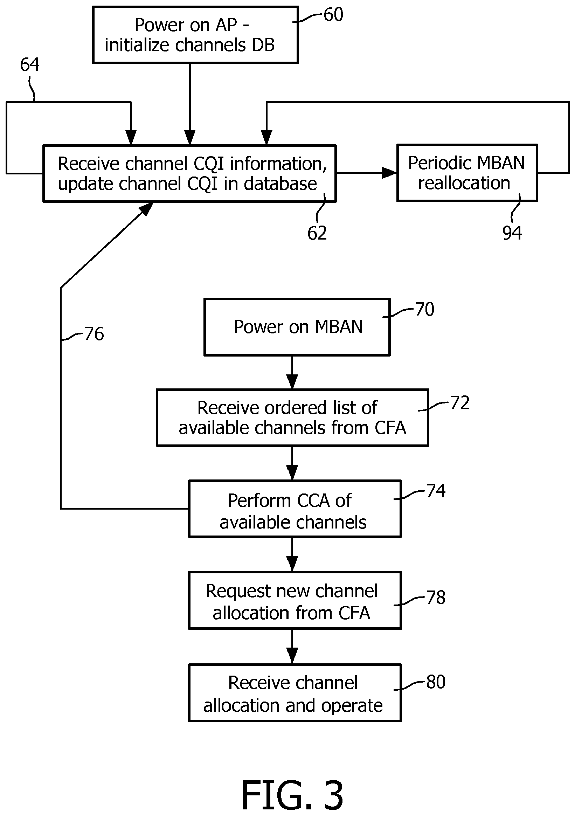

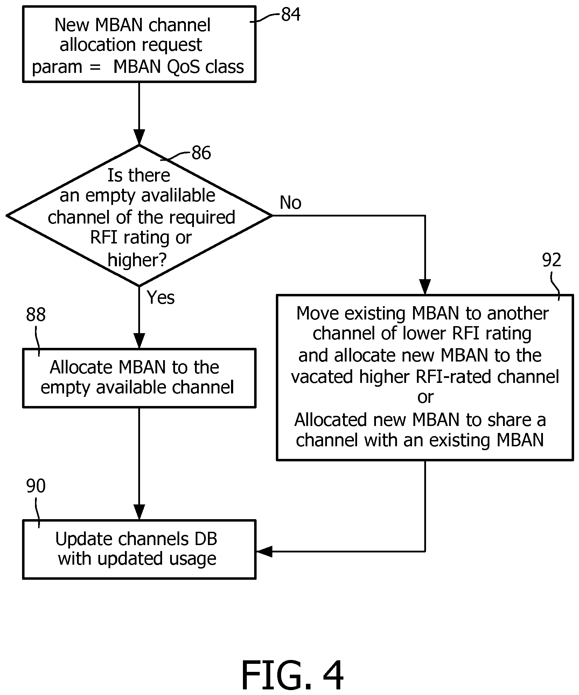

Having disclosed suitable embodiments of the centralized frequency agility system with reference to FIGURES 1 and 2, some further operational aspects are set forth with further reference to the flowcharts of FIGURES 3 and 4.

With reference to FIGURES 1 and 3, startup procedures for initially powering up the AP **20** and the MBAN **10** are set forth. When the AP **20** is powered on, its CFA sub-system **40** and associated channels database **48** are initialized in an operation **60.** The ordered list of available channels **50** is also suitably generated is based on predefined channel RFI ratings. The channel usage status table is initialized by setting all the available channels as 'IDLE'. In an operation **62,** channel CQI information conveyed to the AP **20** from the MBAN systems **10, 35, 36** and/or the monitoring device(s) **44** via the longer range communication are used to initialize or update the channel CQI values in the channels database **48,** and the cumulative information in the channels database **48** is used to allocate MBAN systems to available channels having channel CQI compatible with the MBAN QoS classes. The operation **62** is updated as additional channel CQI information is received, as indicated by the loop **64.**

The operation **62** is performed in conjunction with CCA or other CQI information acquisition performed by the MBAN systems **10, 35, 36** and/or the monitoring device(s) **44,** as diagrammatically shown for the illustrative MBAN system **10.** In FIGURE 3, the MBAN **10** powers up in an operation **70** and receives the ordered list **50** of channels via the longer range communication in an operation **72.** The MBAN system **10** then performs CCA or other channel CQI information acquisition in an operation **74,** and the CQI information is conveyed to the CFA sub-system **40** via the longer range communication for use in the operation **62** as diagrammatically indicated in FIGURE 3 by connecting arrow **76.** In an operation **78** the MBAN system **10** sends a request for new channel allocation to the CFA sub-system **40** via the longer range communicaiton, and in an operation **80** the MBAN system **10** receives the new channel allocation from the CFA sub-system **40,** again via the longer range communicaiton, and begins MBAN operation.

With continuing reference to FIGURES 1 and 3 and with further reference to FIGURE 4, the operation **78** generates a new MBAN channel allocation request **84** that is processed by the CFA sub-system **40** as shown in FIGURE 4. The new MBAN channel allocation request **84** has an associated MBAN class parameter indicating the MBAN QoS class **46** of the MBAN **10** for which the new channel is to be allocated. In an operation **86,** the CFA sub-system **40** searches the channels database **48** for an empty available channel. In this context, 'empty' means the channel status information in the usage status table is 'Idle' and also the CCA **74** performed by the MBAN **10** also showed the channel to be 'idle'. If the operation **86** identifies an empty available channel, then the CFA sub-system **40** allocates the MBAN **10** to that channel in an operation **88,** and sends the channel allocation response to the hub device **14** of the MBAN **10** with the selected empty channel number. The MBAN **10** begins operations at the allocated channel (this corresponds to the receipt-and-operate operation **80** performed at the MBAN **10**), and the MBAN **10** optionally sends back a channel assignment confirmation to CFA sub-system **40.** The CFA sub-system **40** updates the channel database **48** with the new channel assignment in an operation **90.** On the other hand, if there is no 'empty' channel available, then the CFA sub-system **40** performs an operation **92** in which it searchs for a 'busy' channel in the channel database **48** that is in use by an existing MBAN system having a lower QoS class than the new MBAN system **10.** If such a 'busy' channel is found, the CFA sub-system **40** sends commands to the MBAN system operating on that 'busy channel and reallocates them to other channels of lower (but still acceptable) channel CQI, and the CFA sub-system **40** allocated the MBAN system **10** to the vacated channel and follows up with the database update of operation **90.** The 'other channel' to which the pre-existing MBAN system of lower MBAN QoS class is reallocated could be a 'busy' channel already in use by one or more other MBAN systems, so long as the sum of their aggregated duty cycles is lower than some threshold so as to guarantee that there is no significant collision probability increase caused by the reallocation. In the extreme case that there are too many MBAN systems crowded together, the CFA sub-system **40** can generate a warning message to system administrators.

When an active MBAN system moves into the service area of the AP **20,** it will handover and connect to the AP **20.** This MBAN system suitably continues to work on its current short-range wireless communication channel, but also reports its current channel allocation, its MBAN QoS class, and its aggregated duty cycle to the CFA sub-system **40** of the AP **20.** The CFA sub-system **40** determines whether the new MBAN system operating on its current channel could cause potential collision increase in that channel. If no, then the CFA sub-system **40** updates the channels database **48** to reflect usage of the channel by the newly entrant MBAN system. On the other hand, if the collision probability is increased, the channel has an RFI rating indicative of a high likelihood of RFI, or is otherwise not acceptable, then the CFA sub-system **40** performs the process of FIGURE 4 to allocate a new channel to the newly entrant MBAN system.

If an MBAN system detects channel quality degradation and cannot work properly, for example due to non-MBAN RFI or collision with short-range wireless communication of a nearby MBAN system on the same channel, then the MBAN system suitably makes a local channel reallocation so as to switch to a new channel. This local channel reallocation is suitably based on the CCA performed by the MBAN system and based on the copy of the ordered list of available channels **50** stored at the MBAN system. Such local channel reallocation ensures that an MBAN system can quickly switch to a new channel, and can thereby avoid loss of potentially critical medical data. However, the local channel reallocation is provisional. The MBAN system reports the local channel reallocation to the CFA sub-system **40,** which determines whether the local channel reallocation is acceptable based on the information contained in the centralized channels dataabase **48.** If the local channel reallocation is not acceptable, then the CFA sub-system **40** performs the process of FIGURE 4 to allocate a new channel to the MBAN system, so as to effectively "overrule" the local channel reallocation.

With continuing reference to FIGURE 1 and with further reference back to FIGURE 3, the CFA sub-system **40** optionally performs a periodic MBAN system reallocation operation **94.** This operation is performed is a centralized update of the MBAN system channel allocations, and can (by way of example) move the MBAN systems having the highest MBAN QoS class to the best quality channels (as measured by current channel CQI and channel RFI rating) and switch MBAN systems having lower MBAN QoS class(es) to other available channels of lower quality. The periodic reallocation operation **94** ensures that MBAN systems are optimally allocated amongst the available channels.

When an MBAN system moves out the serving area of the AP **20,** or when an MBAN system served by the AP **20** is turned off, then the CFA sub-system **40** of the AP **20** suitably removes the channel usage information for that MBAN system from the channels database **48.**

This application has described one or more preferred embodiments. Modifications and alterations may occur to others upon reading and understanding the preceding detailed description. It is intended that the application be construed as including all such modifications and alterations insofar as they come within the scope of the appended claims.

## Claims

1. A medical system comprising:
a plurality of medical body area network, MBAN, systems (10, 35, 36), each MBAN system (10) comprising a plurality of network nodes (12, 14), said nodes arranged to acquire patient data and intercommunicate via short-range wireless communication;
a central network (20, 22, 23, 24) arranged to communicate with the MBAN systems via longer range communication that is of longer range than the short-range wireless communication; and
a central frequency agility sub-system (40) configured to communicate with the MBAN systems, the central frequency agility sub-system arranged to receive current channel quality information for a plurality of available channels for the short-range wireless communication and allocate the MBAN systems amongst the available channels based at least on the received current channel quality information, on radio frequency interference ratings being indicative for the likelihood of non-MBAN radio frequency interference on each of the plurality of available channels and on quality of service, QoS, classifications (46) of the MBAN systems related to the criticality of the patient data acquired by each of the plurality of MBAN systems.

2. The medical system as set forth in claim 1, wherein each MBAN system (10) includes a plurality of network nodes (12) communicating with a hub device (14) via short-range wireless communication, the hub device communicating with the central network (20, 22, 23, 24) via the longer range communication.

3. The medical system as set forth in any one of claims 1-2, wherein the criticality of the patient data is defined by a pre-assigned quality of service classification (46) to the MBAN system, wherein the highest quality of service is assigned to the MBAN systems engaged in life-critical applications and the lowest quality of service is assigned to the MBAN systems engaged in non-critical applications such as fitness monitoring..

4. The medical system as set forth in claim 3, wherein responsive to receiving a new channel allocation request from an unallocated MBAN system (10) the central frequency agility sub-system (40) is configured to perform a method comprising:
allocating the unallocated MBAN system to an available channel that is empty conditional on there being an empty available channel having a radio frequency interference rating compatible with a quality of service classification of the unallocated MBAN system, and
if there are no empty available channels having a radio frequency interference rating compatible with the quality of service classification of the unallocated MBAN system, then reallocating an already-operating MBAN system having a lower quality of service classification than the quality of service classification of the unallocated MBAN system to another channel and allocating the unallocated MBAN system to the channel vacated by the reallocating.

5. The medical system as set forth in any one of claims 3-4, wherein the central frequency agility sub-system (40) is configured to assign:
a radio frequency interference rating indicative of a relatively lower likelihood of radio frequency interference to channels exclusively assigned for MBAN system short-range wireless communication, and
a radio frequency interference rating indicative of a relatively higher likelihood of radio frequency interference to channels having shared assignment to both MBAN system short-range wireless communication and at least one type of non-MBAN short-range wireless communication.

6. The medical system as set forth in any one of claims 1-5, wherein the MBAN systems (10, 35, 36) are configured to:
acquire current channel quality information for the plurality of available channels, and
send the acquired current channel quality information to the central frequency agility sub-system (40) via the longer range communication.

7. The medical system as set forth in any one of claims 1-6, further comprising: at least one spectrum monitoring device (44) configured to:
acquire current channel quality information for the plurality of available channels, and
send the acquired current channel quality information to the central frequency agility sub-system (40) via the longer range communication.

8. The medical system as set forth in any one of claims 1-7, wherein the central frequency agility sub-system (40) is configured to:
construct an ordered list (50) of available channels that is sorted at least on current channel quality information for the available channels, and
send the ordered list of available channels to the plurality of MBAN systems (10, 35, 36) via the longer range communication.

9. The medical system as set forth in claim 8, wherein the central frequency agility sub-system (40) is configured to omit from the ordered list (50) of available channels any channel that has current channel quality information indicating the current channel quality is too poor to be used by any MBAN system (10, 35, 36) and/or
any channel that is available to the MBAN systems (10, 35, 36) on a secondary basis and is currently in use by a primary non-MBAN user.

10. The medical system as set forth in any one of claims 8-9, wherein the MBAN systems (10, 35, 36) are configured to:
acquire current channel quality information for only the available channels listed in the ordered list (50) of available channels, and
send the acquired current channel quality information to the central frequency agility sub-system (40) via the longer range communication.

11. The medical system as set forth in any one of claims 8-10, wherein responsive to an MBAN system detecting radio frequency interference or a collision:
the MBAN system (10) is configured to allocate to itself a new channel selected from the ordered list (50) of available channels wherein the selection of the new channel is based at least in part on the ordering of the ordered list,
the MBAN system is configured to communicate the new channel allocation to the central frequency agility sub-system (40), and
the central frequency agility sub-system is configured to accept or override the new channel allocation.

12. The medical system as set forth in any one of claims 8-11, wherein the central frequency agility sub-system (40) is configured to sort the ordered list (50) of available channels based current channel quality information for the available channels and based on radio frequency interference ratings for the channels.

13. The medical system as set forth in any one of claims 1-12, wherein:
the central network (20, 22, 23, 24) includes a longer range wireless communication implemented by a plurality of spatially distributed access points (20, 23, 24); and
the central frequency agility sub-system (40) is configured to allocate the MBAN systems (10, 35, 36) allocated to a common access point (20) amongst the available channels.

14. The medical system as set forth in any one of claims 1-13, wherein the central frequency agility sub-system (40) is configured to periodically repeat the allocating of the MBAN systems (10, 35, 36) amongst the available channels based at least on the current channel quality information and on radio frequency interference ratings being indicative for the likelihood of non-MBAN radio frequency interference on the same channel.

15. A method for channel allocation in a medical system comprising a plurality of medical body area network, MBAN, systems (10, 35, 36), each MBAN system (10) comprising a plurality of network nodes (12, 14), said nodes arranged to acquire patient data and intercommunicate via short-range wireless communication; and a central network (20, 22, 23, 24) arranged to communicate with the MBAN systems via longer range communication that is of longer range than the short-range wireless communication, said method comprising:
collecting current channel quality information for a plurality of channels available to the plurality of MBAN systems (10, 35, 36) for short-range communication amongst the network nodes (12, 14) of the MBAN systems; and
allocating the MBAN systems amongst the plurality of available channels based at least on the collected current channel quality information, on radio frequency interference ratings being indicative for the likelihood of non-MBAN radio frequency interference on each of the plurality of available channels and on quality of service, QoS, classifications (46) of the MBAN systems related to the criticality of the patient data acquired by each of the plurality of MBAN systems.

## Patentansprüche

1. Medizinisches System, umfassend:
mehrere medizinische, körpernahe Netzwerk-, MBAN, Systeme (10, 35, 36), wobei jedes MBAN-System (10) eine Vielzahl von Netzwerkknoten (12, 14) umfasst, wobei die Knoten so eingerichtet sind, dass sie Patientendaten erfassen und über drahtlose Nahbereichskommunikation miteinander kommunizieren;
ein zentrales Netzwerk (20, 22, 23, 24), das so eingerichtet ist, dass es mit den MBAN-Systemen über Kommunikation mit größerer Reichweite kommuniziert, die über eine größere Reichweite als die drahtlose Nahbereichskommunikation stattfindet; sowie
ein zentrales Frequency-Agility-Subsystem (40), das so konfiguriert ist, dass es mit den MBAN-Systemen kommuniziert, wobei das zentrale Frequency-Agility-Subsystem so eingerichtet ist, dass es aktuelle Kanalgüteinformationen für mehrere verfügbare Kanäle zur drahtlosen Nahbereichskommunikation empfängt und die MBAN-Systeme aufgrund von zumindest den empfangenen, aktuellen Kanalgüteinformationen, Funkfrequenzinterferenzbemessungen, die für die Wahrscheinlichkeit von Nicht-MBAN-Funkfrequenzinterferenz auf jedem der mehreren verfügbaren Kanäle bezeichnend sind, sowie Dienstgüte-, QoS-, Klassifizierungen (46) der MBAN-Systeme, die auf die Kritikalität der von jedem der mehreren MBAN-Systeme erfassten Patientendaten bezogen sind, unter den verfügbaren Kanälen zuordnet.

2. Medizinisches System nach Anspruch 1, wobei jedes MBAN-System (10) eine Vielzahl von Netzwerkknoten (12) enthält, die mit einer Netzknotenvorrichtung (14) über drahtlose Nahbereichskommunikation kommunizieren, wobei die Netzknotenvorrichtung mit dem zentralen Netzwerk (20, 22, 23, 24) über die Kommunikation mit größerer Reichweite kommuniziert.

3. Medizinisches System nach einem der Ansprüche 1-2, wobei die Kritikalität der Patientendaten durch eine dem MBAN-System vorher zugewiesene Dienstgüteklassifizierung (46) definiert wird, wobei die höchste Dienstgüte den mit lebenskritischen Applikationen beschäftigten MBAN-Systemen zugewiesen wird und die geringste Dienstgüte den mit nicht-kritischen Applikationen beschäftigten MBAN-Systemen, wie zum Beispiel Fitness-Überwachung, zugewiesen wird.

4. Medizinisches System nach Anspruch 3, wobei, in Reaktion auf das Empfangen einer neuen Kanalzuweisungsanforderung von einem nicht zugewiesenen MBAN-System (10), das zentrale Frequency-Agility-Subsystem (40) so konfiguriert ist, dass es ein Verfahren durchführt, wonach:
das nicht zugewiesene MBAN-System einem verfügbaren Kanal zugewiesen wird, der frei ist, unter der Voraussetzung, dass es sich um einen freien, verfügbaren Kanal mit einer Funkfrequenzinterferenzbemessung handelt, die mit einer Dienstgüteklassifizierung des nicht zugewiesenen MBAN-Systems kompatibel ist, und
wenn keine freien, verfügbaren Kanäle mit einer Funkfrequenzinterferenzbemessung, die mit der Dienstgüteklassifizierung des nicht zugewiesenen MBAN-Systems kompatibel ist, verfügbar sind, ein bereits arbeitendes MBAN-System mit einer geringeren Dienstgüteklassifizierung als der Dienstgüteklassifizierung des nicht zugewiesenen MBAN-Systems dann einem anderen Kanal neu zugewiesen wird und das nicht zugewiesene MBAN-System dem durch die Neuzuweisung frei gewordenen Kanal zugewiesen wird.

5. Medizinisches System nach einem der Ansprüche 3-4, wobei das zentrale Frequency-Agility-Subsystem (40) so konfiguriert ist, dass es zuordnet:
eine Funkfrequenzinterferenzbemessung, die für eine relativ geringere Funkfrequenzinterferenzwahrscheinlichkeit bezeichnend ist, zu Kanälen, die ausschließlich der drahtlosen MBAN-System-Nahbereichskommunikation zugeordnet sind, und
eine Funkfrequenzinterferenzbemessung, die für eine relativ höhere Funkfrequenzinterferenzwahrscheinlichkeit bezeichnend ist, zu Kanälen mit geteilter Zuordnung zu sowohl der drahtlosen MBAN-System-Nahbereichskommunikation als auch zu mindestens einer Art der drahtlosen Nicht-MBAN-Nahbereichskommunikation.

6. Medizinisches System nach einem der Ansprüche 1-5, wobei die MBAN-Systeme (10, 35, 36) so konfiguriert sind, dass sie:
aktuelle Kanalgüteinformationen für die mehreren verfügbaren Kanäle erfassen, und
die erfassten, aktuellen Kanalgüteinformationen über die Kommunikation mit größerer Reichweite zu dem zentralen Frequency-Agility-Subsystem (40) übertragen.

7. Medizinisches System nach einem der Ansprüche 1-6, weiterhin umfassend:
mindestens eine Spektrumsüberwachungsvorrichtung (44), die so konfiguriert ist, dass sie:
aktuelle Kanalgüteinformationen für die mehreren verfügbaren Kanäle erfasst, und
die erfassten, aktuellen Kanalgüteinformationen über die Kommunikation mit größerer Reichweite zu dem zentralen Frequency-Agility-Subsystem (40) überträgt.

8. Medizinisches System nach einem der Ansprüche 1-7, wobei das zentrale Frequency-Agility-Subsystem (40) so konfiguriert ist, dass es:
eine geordnete Liste (50) von verfügbaren Kanälen erstellt, die zumindest nach aktuellen Kanalgüteinformationen für die verfügbaren Kanäle geordnet ist, und
die geordnete Liste von verfügbaren Kanälen über die Kommunikation mit größerer Reichweite zu den mehreren MBAN-Systemen (10, 35, 36) überträgt.

9. Medizinisches System nach Anspruch 8, wobei das zentrale Frequency-Agility-Subsystem (40) so konfiguriert ist, dass es aus der geordneten Liste (50) von verfügbaren Kanälen einen Kanal, der aktuelle Kanalgüteinformationen aufweist, die angeben, dass die aktuelle Kanalgüte zu schlecht ist, um von einem MBAN-System (10, 35, 36) benutzt zu werden, und/oder einen Kanal, der den MBAN-Systemen (10, 35, 36) auf sekundärer Basis zur Verfügung steht und aktuell von einem primären Nicht-MBAN-Nutzer genutzt wird, auslässt.

10. Medizinisches System nach einem der Ansprüche 8-9, wobei die MBAN-Systeme (10, 35, 36) so konfiguriert sind, dass sie:
aktuelle Kanalgüteinformationen für lediglich die in der geordneten Liste (50) von verfügbaren Kanälen aufgeführten, verfügbaren Kanäle erfassen, und
die erfassten, aktuellen Kanalgüteinformationen zu dem zentralen Frequency-Agility-Subsystem (40) über die Kommunikation mit größerer Reichweite übertragen.

11. Medizinisches System nach einem der Ansprüche 8-10, wobei, in Reaktion darauf, dass ein MBAN-System eine Funkfrequenzinterferenz oder eine Kollision detektiert:
das MBAN-System (10) so konfiguriert ist, dass es sich einen aus der geordneten Liste (50) von verfügbaren Kanälen ausgewählten, neuen Kanal zuweist, wobei die Wahl des neuen Kanals zumindest teilweise auf der Ordnung der geordneten Liste basiert,
das MBAN-System so konfiguriert ist, dass es dem zentralen Frequency-Agility-Subsystem (40) die neue Kanalzuweisung mitteilt, und
das zentrale Frequency-Agility-Subsystem so konfiguriert ist, dass es die neue Kanalzuweisung akzeptiert oder aufhebt.

12. Medizinisches System nach einem der Ansprüche 8-11, wobei das zentrale Frequency-Agility-Subsystem (40) so konfiguriert ist, dass es die geordnete Liste (50) von verfügbaren Kanälen auf der Basis von aktuellen Kanalgüteinformationen für die verfügbaren Kanäle und auf der Basis von Funkfrequenzinterferenzbemessungen für die Kanäle sortiert.

13. Medizinisches System nach einem der Ansprüche 1-12, wobei:
das zentrale Netzwerk (20, 22, 23, 24) eine durch mehrere räumlich verteilte Zugriffspunkte (20, 23, 24) implementierte, drahtlose Kommunikation mit größerer Reichweite vorsieht;
und das zentrale Frequency-Agility-Subsystem (40) so konfiguriert ist, dass es die zugewiesenen MBAN-Systeme (10, 35, 36) einem gemeinsamen Zugriffspunkt (20) unter den verfügbaren Kanälen zuweist.

14. Medizinisches System nach einem der Ansprüche 1-13, wobei das zentrale Frequency-Agility-Subsystem (40) so konfiguriert ist, dass es das Zuweisen der MBAN-Systeme (10, 35, 36) unter den verfügbaren Kanälen aufgrund von zumindest den aktuellen Kanalgüteinformationen sowie Funkfrequenzinterferenzbemessungen, die für die Wahrscheinlichkeit von Nicht-MBAN-Funkfrequenzinterferenz auf dem gleichen Kanal bezeichnend sind, periodisch wiederholt.

15. Verfahren zur Kanalzuweisung in einem medizinischen System, umfassend mehrere medizinische, körpernahe Netzwerk-, MBAN, Systeme (10, 35, 36), wobei jedes MBAN-System (10) eine Vielzahl von Netzwerkknoten (12, 14) umfasst, wobei die Knoten so eingerichtet sind, dass sie Patientendaten erfassen und über drahtlose Nahbereichskommunikation miteinander kommunizieren; sowie ein zentrales Netzwerk (20, 22, 23, 24), das so eingerichtet ist, dass es mit den MBAN-Systemen über Kommunikation mit größerer Reichweite kommuniziert, die über eine größere Reichweite als die drahtlose Nahbereichskommunikation stattfindet, wobei das Verfahren die folgenden Schritte umfasst, wonach:
aktuelle Kanalgüteinformationen für mehrere Kanäle erfasst werden, die den mehreren MBAN-Systemen (10, 35, 36) zur Nahbereichskommunikation unter den Netzwerkknoten (12, 14) der MBAN-Systeme zur Verfügung stehen; und
die MBAN-Systeme unter den mehreren verfügbaren Kanälen aufgrund von zumindest den erfassten, aktuellen Kanalgüteinformationen, Funkfrequenzinterferenzbemessungen, die für die Wahrscheinlichkeit von Nicht-MBAN-Funkfrequenzinterferenz auf jedem der mehreren verfügbaren Kanäle bezeichnend sind, sowie Dienstgüte-, QoS-, Klassifizierungen (46) der MBAN-Systeme, die auf die Kritikalität der von jedem der mehreren MBAN-Systeme erfassten Patientendaten bezogen sind, zugeordnet werden.

## Revendications

1. Système médical comprenant :
une pluralité de systèmes (10, 35, 36) de réseau corporel médical (MBAN), chaque système MBAN (10) comprenant une pluralité de noeuds de réseau (12, 14), lesdits noeuds étant agencés de manière à acquérir des données de patient et communiquer entre eux par l'intermédiaire d'une communication sans fil de courte portée ;
un réseau central (20, 22, 23, 24) agencé de manière à communiquer avec les systèmes MBAN par l'intermédiaire d'une communication de plus longue portée qui est d'une portée plus longue que celle de la communication sans fil de courte portée ; et
un sous-système d'agilité de fréquence central (40) conçu pour communiquer avec les systèmes MBAN, le sous-système d'agilité de fréquence central étant agencé de manière à recevoir des informations de qualité de canal courantes pour une pluralité de canaux disponibles pour la communication sans fil de courte portée et affecter les systèmes MBAN parmi les canaux disponibles en fonction au moins des informations de qualité de canal courantes reçues, de notes de brouillage radiofréquence indicatives de la probabilité de brouillage radiofréquence non MBAN sur chacun de la pluralité de canaux disponibles, et de classements (46) de qualité de service (QoS) des systèmes MBAN relatifs à la criticité des données de patient acquises par chacun de la pluralité de systèmes MBAN.

2. Système médical selon la revendication 1, dans lequel chaque système MBAN (10) comprend une pluralité de noeuds de réseau (12) communiquant avec un dispositif concentrateur (14) par l'intermédiaire d'une communication sans fil de courte portée, le dispositif concentrateur communiquant avec le réseau central (20, 22, 23, 24) par l'intermédiaire de la communication de plus longue portée.

3. Système médical selon l'une quelconque des revendications 1 et 2, dans lequel la criticité des données de patient est prédéfinie par un classement de qualité de service (46) préalablement attribué au système MBAN, dans lequel la qualité de service la plus élevée est attribuée aux systèmes MBAN engagés dans des applications vitales et la qualité de service la plus faible est attribuée aux systèmes MBAN engagés dans des applications non vitales telles que la surveillance de la condition physique.

4. Système médical selon la revendication 3, dans lequel en réponse à la réception d'une nouvelle demande d'affectation de canal émanant d'un système MBAN (10) non affecté, le sous-système d'agilité de fréquence central (40) est conçu pour exécuter un procédé comprenant :
l'affectation du système MBAN non affecté à un canal disponible qui est vide, à condition qu'il existe un canal disponible vide ayant une note de brouillage radiofréquence compatible avec un classement de qualité de service du système MBAN non affecté, et
s'il n'existe aucun canal disponible ayant une note de brouillage radiofréquence compatible avec le classement de qualité de service du système MBAN non affecté, la réaffectation d'un système MBAN déjà utilisé ayant un classement de qualité de service inférieur au classement de qualité de service du système MBAN non affecté à un autre canal et l'affectation du système MBAN non affecté au canal rendu vacant par la réaffectation.

5. Système médical selon l'une quelconque des revendications 3 et 4, dans lequel le sous-système d'agilité de fréquence central (40) est conçu pour attribuer :
une note de brouillage radiofréquence indicative d'une probabilité relativement faible de brouillage radiofréquence aux canaux affectés exclusivement à la communication sans fil de courte portée de systèmes MBAN, et
une note de brouillage radiofréquence indicative d'une probabilité relativement élevée de brouillage radiofréquence aux canaux ayant une affectation commune aussi bien à une communication sans fil de courte portée de systèmes MBAN qu'à au moins un type de communication sans fil de courte portée non MBAN.

6. Système médical selon l'une quelconque des revendications 1 à 5, dans lequel les systèmes MBAN (10, 35, 36) sont conçus pour :
acquérir des informations de qualité de canal courantes pour la pluralité de canaux disponibles, et
envoyer les informations de qualité de canal courantes acquises au sous-système d'agilité de fréquence central (40) par l'intermédiaire de la communication de plus longue portée.

7. Système médical selon l'une quelconque des revendications 1 à 6, comprenant en outre :
au moins un dispositif de surveillance de spectre (44) conçu pour :
acquérir des informations de qualité de canal courantes pour la pluralité de canaux disponibles, et
envoyer les informations de qualité de canal courantes acquises au sous-système d'agilité de fréquence central (40) par l'intermédiaire de la communication de plus longue portée.

8. Système médical selon l'une quelconque des revendications 1 à 7, dans lequel le sous-système d'agilité de fréquence central (40) est conçu pour :
constituer une liste ordonnée (50) de canaux disponibles qui est triée au moins sur des informations de qualité de canal courantes pour les canaux disponibles, et
envoyer la liste ordonnée de canaux disponibles à la pluralité de systèmes MBAN (10, 35, 36) par l'intermédiaire de la communication de plus longue portée.

9. Système médical selon la revendication 8, dans lequel le sous-système d'agilité de fréquence central (40) est conçu pour ne pas inclure dans la liste ordonnée (50) de canaux disponibles un canal dont les informations de qualité de canal courantes indiquent que la qualité de canal courante est trop mauvaise pour permettre son utilisation par un système MBAN (10, 35, 36) et/ou
un canal qui est disponible pour les systèmes MBAN (10, 35, 36) à titre secondaire et qui est actuellement utilisé par un utilisateur primaire non MBAN.

10. Système médical selon l'une quelconque des revendications 8 et 9, dans lequel les systèmes MBAN (10, 35, 36) sont conçus pour :
acquérir des informations de qualité de canal courantes seulement pour les canaux disponibles recensés dans la liste ordonnée (50) de canaux disponibles, et
envoyer les informations de qualité de canal courantes acquises au sous-système d'agilité de fréquence central (40) par l'intermédiaire de la communication de plus longue portée.

11. Système médical selon l'une quelconque des revendications 8 à 10, dans lequel en réponse à la détection par le système MBAN d'un brouillage radiofréquence ou d'une collision :
le système MBAN (10) est conçu pour s'affecter un nouveau canal choisi dans la liste ordonnée (50) de canaux disponibles, le choix du nouveau canal étant fonction au moins en partie de l'ordre établi dans la liste ordonnée,
le système MBAN est conçu pour transmettre la nouvelle affectation de canal au sous-système d'agilité de fréquence central (40), et
le sous-système d'agilité de fréquence central est conçu pour accepter la nouvelle affectation de canal ou pour l'ignorer.

12. Système médical selon l'une quelconque des revendications 8 à 11, dans lequel le sous-système d'agilité de fréquence central (40) est conçu pour trier la liste ordonnée (50) de canaux disponibles en fonction d'informations de qualité de canal courantes pour les canaux disponibles et en fonction de notes de brouillage radiofréquence pour les canaux.

13. Système médical selon l'une quelconque des revendications 1 à 12, dans lequel :
le réseau central (20, 22, 23, 24) comprend une communication sans fil de plus longue portée mise en oeuvre par une pluralité de points d'accès (20, 23, 24) répartis dans l'espace ; et
le sous-système d'agilité de fréquence central (40) est conçu pour affecter les systèmes MBAN (10, 35, 36) affectés à un point d'accès commun (20) parmi les canaux disponibles.

14. Système médical selon l'une quelconque des revendications 1 à 13, dans lequel le sous-système d'agilité de fréquence central (40) est conçu pour répéter périodiquement l'affectation des systèmes MBAN (10, 35, 36) parmi les canaux disponibles en fonction au moins des informations de qualité de canal courantes et de notes de brouillage radiofréquence indicatives de la probabilité de brouillage radiofréquence non MBAN sur le même canal.

15. Procédé d'affectation de canal dans un système médical comprenant une pluralité de systèmes (10, 35, 36) de réseau corporel médical (MBAN), chaque système MBAN (10) comprenant une pluralité de noeuds de réseau (12, 14), lesdits noeuds étant agencés de manière à acquérir des données de patient et communiquer entre eux par l'intermédiaire d'une communication sans fil de courte portée ; et un réseau central (20, 22, 23, 24) agencé de manière à communiquer avec les systèmes MBAN par l'intermédiaire d'une communication de plus longue portée qui est d'une portée plus longue que celle de la communication sans fil de courte portée, ledit procédé comprenant :
la collecte d'informations de qualité de canal courantes pour une pluralité de canaux disponibles pour la pluralité de systèmes MBAN (10, 35, 36) pour une communication de courte portée parmi les noeuds de réseau (12, 14) des systèmes MBAN ; et
l'affectation des systèmes MBAN parmi la pluralité de canaux disponibles en fonction au moins des informations de qualité de canal courantes collectées, de notes de brouillage radiofréquence indicatives de la probabilité de brouillage radiofréquence non MBAN sur chacun de la pluralité de canaux disponibles et de classements (46) de qualité de service (QoS) des systèmes MBAN relatifs à la criticité des données de patient acquises par chacun de la pluralité de systèmes MBAN.
